# EUROPEAN PATENT APPLICATION

(11) **EP 4 345 827 A1**
(43) Date of publication of application: **03.04.2024**
(21) Application number: 22020475.4
(22) Date of filing: 30.09.2022
(51) Int. Cl.: G16H 10/60, G16H 40/00, G16H 50/20, G16H 80/00

(54) **A PROTOTYPE METHOD FOR THE COMMUNICATION AND CONTINUOUS MONITORING OF THE PHYSICAL AND EMOTIONAL STATE OF THE PATIENTS THROUGH A BEDSIDE SYSTEM**

(71) Applicant: BioAssist S.A., 26504 Rio, Achaia (GR)
(72) Inventor: The designation of the inventor has not yet been filed

(57) **Abstract**

A method of providing care for patients whose lives are intertwined with their care bed. The method, which is implemented through the prototype system "SISEI", manages to remove limitations and shortcomings of the existing technique, exploiting emerging technologies from the field of Digital Health, Computer Vision and Affective Computing. The method integrates original innovative functions into a single system, which can combine the provided services to draw useful conclusions about the patient's health course and allows the assimilation of new technologies due to the architecture based on web services. The method's innovation is suggested in the pioneering combined fulfillment of all the emerged deficiencies by the existing bedside systems of the healthcare, which is enriched, at the same time, with secondary original services for the evaluation of the emotional state as well as of the mental and physical exercise.

## Description

The current invention relates to a communication and monitoring method through a bedside system that allows continuous assessment of the physical and emotional state of the hospitalized patients.

Currently, bedside systems are being developed either as medical informatics systems to monitor the patient's condition by integrating technologies such as Electronic Health Records and electronic recording of bio signals, either as commercial infotainment solutions that provide video calling, gaming, movie, and TV viewing as well as information via internet access. Overall, they use hardware (arms-screens-sensors), which is made for the respective system or general-purpose devices (tablets, mobiles) that provide integrated the required technologies, while the developing software aims to meet the requirements set by the assumed title (commercial infotainment systems / medical monitoring systems). During the review of the relevant scientific literature, there are no cases of integration of the two different types in an information system that implements all the functions.

Concurrently, with the development of a new field of computing that is referred in the literature as Affective Computing and is dealing with the interpretation, recognition, analysis, and quantification of human emotion during its interaction with machines (Human-Computer Interaction), a generated need is to integrate emotional computing functionality into healthcare delivery information systems. The main incentives are based on the need for empathy of the medical staff and the patient's relatives, as well as the prevention of dangerous situations that are directly related to the emotional state of the patient. Affective computing has already been applied in patient healthcare computing fields such as self-sufficient systems for managing patient breathing using Kinect, for analyzing emotion from facial expressions in Alzheimer's patients, and for detecting Parkinson's through inability to express specific emotions. However, absent from the literature is a bedside system that integrates emotional computing functionality to identify patients' psychological transitions in real time and provide timely intervention by competent staff or the patient's social circle.

In the context of advanced capabilities development during human-machine interaction, the field of Exergames has been created in the IT industry. By using the human figure or parts of it to provide commands, these games encourage the user to engage physically or mentally in their development. Numerous studies emphasize the use of Exergames, which provide a variety of benefits regarding the mental and physical health of patients, elderly, children as well as people with disabilities. An essential requirement in most cases is the use of platforms (gaming machines) connected to peripheral subsystems, which carry expensive advanced sensors with the main purpose of detecting the human figure in a place. This necessity is a crucial impediment for the rapid spread of similar systems in domestic environments as well as in areas of health interest.

As far as bedside care delivery systems connected to bio signals measurement sensor devices for patient monitoring, it should be highlighted that their development is greatly hampered by problems related to the communication of the peripheral sensor devices with the reporting platform that manages connections, sensor control and data exchange management. These problems have as their main axis the diversity in available communication protocols, hardware, and data encapsulation standards. Therefore, in the specific technological field, the need to develop a solution that integrates the process of automated connectivity with all kinds of peripheral systems-sensors regardless of protocols, standards, and hardware (built-in interoperability) is evident.

Considering that bedside healthcare delivery systems mainly relate to the patient, whose condition is often accompanied by distresses in moving the limbs or coordinating brain functions with the limbs, the absence of easy command in the systems is a critical limitation on the acceptance of these systems utilization by the main user. Therefore, the requirement of functionality in similar environments is imperative, not only for the pleasure of users, but also for security issues. However, bedside systems are not just about the patient. They are associated also with a group of specialists, who are responsible for providing care as well as with the patient's family environment, which has a major role in the patient's psychological support. The existing bedside systems fail to integrate into their functionality the whole set of involved roles, since they focus unilaterally on the medical point of view or on their social demonstration.

The purpose of the current invention is to eliminate all the above disadvantages and omissions by offering a technical method of patient bedside care that will enable:
1) The combined monitoring of the patient's vital signs with the use of commercial measuring devices that can connect to a PC in real time by all involved personnel from portable or fixed devices (PC, tablet, mobile). The innovation of the system lies in the ability to process measurements from devices [sphygmomanometers, scales, blood glucose meters, activity meters (listed indicatively and restrictively)] operating with different software, communication protocols and operational standards.
2) The broad involvement of the patient with the social environment and the social media.
3) Personalized information on diet, medications, and health routines as well as news and updates.
4) The assessment of his emotional state by exploiting innovative computer vision and machine learning technologies during a teleconsultation with the doctor.
5) The rehabilitation of the patient's physical and mental condition by using genuine games of the type Cognitive - Exergames with the difference that they are handled by parts of the body where is essential to improve movement. Motion capture and processing for game manipulation is accomplished with innovative computer vision technologies, eliminating the need for specialized and costly hardware.
6) The user-friendly command from the patient to the system, considering the patient's mobility problems by exploiting the smart button capabilities.

The invention relates to the technical method of providing health care that includes information, monitoring, updating and entertainment services for patients with transient or chronic diseases, elderly or disabled people, whose lives are intertwined with their care bed (whether it is in the hospital, in clinic, rehabilitation center, or at their home). The described healthcare delivery method is implemented in practice with the development of the information system "SISEI" (Smart Infotainment System with Emotional Intelligence).

When the patient is admitted to a long-term care and rehabilitation unit, all the related data to the patient's medical care are entered into the bedside information system by the competent nurse. The data consists of the patient's medical history, tests, allergies, medicines, medications, eating habits, exercise, and sleep routines. In this way, the personal electronic health record of the patient is created. In addition to the beneficial avoidance of stationery to record the data, the avoidance of errors is achieved in various cases where they are directly transferred to the system, while at the same time it is possible to review the health record by authorized personnel via a desktop computer, tablet and/or mobile phone. In addition, it is possible to display the data in the form of graphs, which offer a comprehensive view of the collected information, the detection of outliers and the drawing of reliable conclusions in contrast to simple recording.

During the rehabilitation phase, the patient is treated in the bed and next to it, the universal tablet is placed attached to an arm which kinesiological serves all the patient's needs. The choice of a general-purpose tablet for the bedside terminal gives flexibility in the choice of the bracket depending on the requirements, as there are several available models in the commerce that are compatible with all tablet sizes. The patient is also given an activity tracker to wear it all the time, while at regular intervals the nursing staff receives measurements from the patient using smart devices (tables / smartphones) connected to a Wi-Fi network.

In particular, it will be possible for each patient to measure the following indicators / parameters:
1) the temperature of the patient (thermometer),
2) the oxygenation of the blood and its pulses (pulse oximeter),
3) the blood pressure of the patient (sphygmomanometer),
4) the heart rate (to detect arrhythmia),
5) the patient's weight and lipid indicators (digital scales with a lipometer),
6) the physical activity (steps, sleep) of the patient (activity tracker),
7) the adequacy of the administered serum,
8) the fullness of the urine collection container,
9) the stay of a (stimulated and possibly demented) patient within the limits of the hospital bed,
10) the quality of night sleep (e.g. deep, light, restless) - especially for patients with difficulty or inability to communicate with the environment.

Therefore, the system provides the possibility of automated or semi-automated recording of the patient's bio signals and activity. It is important to mention that integrating smart devices into the information system ignores their heterogeneity regarding the different software, communication protocols and operational standards they support. Therefore, the system can connect with a multitude of smart devices and is not limited to the already existing ones, greatly enhancing the concept of interoperability.

The recording of the patient's bio signals and daily activity and the storage in a database aims at their further analysis which, in addition to the detection of outliers, also allows the automated detection of extreme values in bio signals and the automated providing of instructions and advice adapted to the special conditions of each hospitalization. In this way, the resting patient is continuously monitored by the bedside system, which can provide personalized advice and prompts for adherence to the medication but also intervene in the event of an emergency.

The attending physician visits the patient at regular intervals. Instead, the bedside terminal system provides the ability of hold the meeting online, discharging the doctor from the obligation to be present, but without depriving the doctor of the option to evaluate the patient's examinations, and physical condition, since the doctor can access them through the mobile or a tablet or a desktop PC. The peculiarity of this terminal system is that through the projected video it assesses the patient's emotion in real time by quantifying voice characteristics and facial expressions using advanced machine learning algorithms. The derived measurements from the assessment of the patient's emotion are stored in the database along with the measurements of bio signals and physical activity and are co-evaluated by the specialized staff. Detecting the patient's low mental mood triggers the system to call a psychologist or a familiar person for the purpose of mental uplifting the patient.

The doctor calls the patient to inform about the health's condition, after the review about the health status through the bio-signals that are automatically collected and stored by the system. The conversation is initialized and during it, the patient's facial expressions are collected in real time and are analyzed by the predictive sentiment analysis model. The results, in addition to being stored in the Electronic Health Record to assess the patient's emotional state, are also presented on the doctor's screen in real time.

The patient is hospitalized to cure a health condition that has impaired the body's movements or the mental clarity. In order to recover, doctors suggest a medication, which includes drugs, exercise program, diet, rest. Additionally, the bedside system intervenes in the treatment with a series of critical games of type cognitive and exer. Cognitive games aim to mentally exercise memory and clarity, while exer games engage parts of the body that are kinetically challenged in manipulating a video game figure to improve physical fitness. The SISEI bedside system enables the patient to practice with these games, while at the same time, as far as Exergames are concerned, they are handled only with the use of a computer camera through innovative computer vision methods and machine learning models. A game's performance is measured by indicators (e.g. user response time, number of errors, etc.) defined per game with the cooperation of the appropriate medical staff and stored along with bio signals, daily activity, and emotional assessment for further utilization by experts.

The results of the game are stored in the Electronic Health Record and the Doctor or physiotherapist is informed, checks the patient's progress, and increases the level of difficulty if it is essential. These results are considered with measurements of bio signals and emotion. In addition to the patient's entertainment, the bedside system turns into a mental coach, improving the patient's overall physical and mental condition.

The patient, due to the illness or lack of familiarity with computers, is struggling to use the tablet. Similar problems may discourage the basic user from using and accepting the system, leading to failure. The integration of the smart button into the bedside system through which the basic functions of using the tablet are carried out facilitates the acceptance of the system by the user and provides him with a quick way to call for help. The smart button is wireless, capable of communicating via Bluetooth / BLE, and incorporates motion detection sensors, as well as the ability to program the push feature, providing the possibility of remote control of the terminal with a limited number of movements. Various implementations of the smart button are commercially available to meet the system requirements.

When an indicator of the patient's health status differentiates from the predetermined threshold, the event should be reported for evaluation and, possibly, for the execution of further nursing and/or medical care actions. Thresholds are set individually for each patient by the attending doctors, through the application of the bedside system. Depending on the label of an indicator as one of the three alarm levels (green, yellow or red), the proper emergency notification mechanism is also activated. At the same time, the patient has the possibility to call or state an emergency with the smart button.

In summary, the technical method combines all the above services in a solid, easy-to-use, and efficient way. At the same time, the tablet can be used to inform the patient about what is happening in the world, to communicate with family, to view and share multimedia material, to give the option of social involvement and remove the unpleasant feeling of isolation.

The bedside system is available to all the involved roles, who are authorized to access the patient's information and are in charge of their medical care, as well as the social environment. However, access to the provided services and system information is limited by the need-to-know principle. The roles that can interact with the system terminals are the following:
1) Patient,
2) Relative of the patient or in general a person who has taken care of the patient,
3) Nurse who visits the wards,
4) Nurse who is on call (Nurses Station),
5) Supervisor of the Nursing Service,
6) Doctor, from various specialties related to the monitoring, treatment, and rehabilitation of patients (such as, for example, physiatrist, cardiologist, neurologist, General practitioner, etc.),
7) Doctor on call,
8) Other Scientific staff, from various specialties related to the monitoring, treatment, and rehabilitation of patients (such as, for example, psychologist, physiotherapist, occupational therapist, etc.),
9) Technical staff in charge of installation procedures, parameterization, and control of the correct operation of the individual parts of the system, without access to the generated data,
10) Administrator, who has access to user account management tools, statistical data, and diagnostic functions of the system.

The original implementation of the current invention is piloted, within the framework of the SISEI research project, in selected recovery and rehabilitation centers, applying the above usage scenario. The development of the SISEI prototype system is broken down into software and hardware.

Regarding the software, it has been developed in a segmented architecture that serves the effortless integration and removal of services and is divided into three individual subsystems. The SISEI" consists of three individual sub-systems, the back-end sub-system, the user interface sub-system (Front-End) and the Core System. The Back end and Core System are cloud computing structures, as shown in Figure 1, providing computing resources on request, while the Front-End is installed on the platforms intended for its use. In more detail, the basic structure of the system (three subsystems) is subdivided into smaller modules (modules), as follows:
1. Front-End
   i. Android App: The application that is installed inside the bedside terminal. The main function of the application, apart from the user interface, is to handle the communication with the sensors via the Bluetooth / BLE protocol. It also integrates the necessary WebRTC libraries for video conferencing.
   ii. Web App: The web application for system users implemented with JavaScript, HTML and Angular framework technologies.
   iii. Helpdesk App: The web application for system administrators and the nurses' station which is implemented with JavaScript and HTML technologies and the Angular framework.
   iv. WebRTC App: The web application that will provide all the necessary video conferencing features. Used by both the Web and the Helpdesk application for video calls between users. The implementation leverages JavaScript and HTML technologies and the Angular framework.
   v. Exergame App: The application for Exergames that works side-by-side with the bedside terminal on a microcomputer (such as Raspberry or NVIDIA Jetson Nano) that interfaces with a camera and monitor or TV. This application is implemented in Python and performs the computer vision and learning processes to control the game and evaluate the patient's performance.
2. Back-End
   i. Authentication Services: Programming interface for online services with user authentication features (common to all front-end applications), so that user logins can be supported, either registered in the system, or using accounts from popular social networks to log in networks (such as Facebook, Twitter, etc.). Also, in the future, the user login process for all system applications can be facilitated with a single login (Single sign-on function). The implementation is based on the popular jS library - Passport.
   ii. API Services: Programming interface for online services that will communicate with the Front-end interfaces of the system, such as the Android application of the bedside terminal and the Web application. The solution is based on the NodeJS platform and the express.js framework.
   iii. Admin API Services: Programming interface for online services related to system management functions, which will communicate with the Helpdesk interface (front-end) of the system. The solution is based on the NodeJS platform and the express.js framework.
   iv. Emotion Analysis Services: Programming interface for web services regarding the analysis of emotions using machine learning models. Specifically, the subsystem analyzes the images and videos sent to it by user applications and evaluates the emotional state by reducing it to 6 basic emotions: sadness, happiness, fear, anger, surprise and disgust. An example of an embedded machine learning model is the use of CNNs - Convolutional Neural Networks trained on the FER-2013 dataset.
   v. Video Recording Services: Programming interface for online services related to the process of recording video calls. The subsystem is implemented in python language with integration of WebRTC library.
   vi. Cron Server: Suitable application (Cron type) for executing jobs in the Background at the appropriate times that will be scheduled. For example, sending emails with references to doctors at a specific time, e.g. once a week. The solution is based on the NodeJS platform and the express.js framework.
   vii. Websocket Server: Application for the operation of Websockets, so that communication between applications is achieved in real time wherever and whenever this is required. The solution is based on the NodeJS platform and the express.js framework.
3. Core System
   i. Database: SQL database required to store all required system information/data. The present implementation is based on the MariaDB database.
   ii. Redis: A system that allows applications that will run in the back-end to communicate directly with each other using cache mechanisms to increase performance.
   iii. coturn: Turn Server to support webRTC communication between applications using the coturn framework in a docker container.

Each sub-unit (module) type of web service implements one by one a conceivable part of the overall system's functionality serving a client-server collaboration based on the REST architectural model. Systems that support the REST model do not know the previous state (stateless) and separate concerns between client and server. This means that knowledge of each other's status is not required, nor is knowledge of previous messages required. Modules are parts of the cloud computing structure. All of them together with the sub-modules (Cron-Websocket Servers) play the role of the server in the client-server cooperation of the system. The role of the client has been assumed by online applications, which request information-data-knowledge from the servers through http requests. About the core of the system, it basically consists of the database, Redis and coturn. The three modules work mutually supportively with Redis, as a cache memory, increasing the overall performance.

As regards the patient side, all the aforementioned features are implemented using hardware that may include the following categories of devices:
1. Tablet
2. Smart button for immediate intervention and remote control
3. Smart watch
4. Sphygmomanometer
5. Scale
6. Oximeter
7. Activity tracker.

The devices communicate with the Android app via Bluetooth / BLE. For the above categories of devices, a wide variety of models are commercially available from many manufacturers and the choice between them depends on the requirements of the clinical structure in which the system will be installed and the medical monitoring needs of the patients. The system can support any model with the ability to communicate via Bluetooth / BLE, providing independence from the respective hardware and allowing the continuous upgrade of the technological infrastructure. The integration of new device models into the system requires the availability of the communication protocol or corresponding mechanism from the manufacturer's side. For the original implementation of the system, at least one model has been integrated for each of the above categories. Examples include the manufacturers iHealth Labs, Beurer, Jumper Medical and Xiaomi, selected models of which have been integrated, while Espruino's puck.js model has been used for the smart button.

## Claims

1. A method of monitoring, informing and entertaining patients in health care and rehabilitation units based on communication and monitoring through a bedside terminal system, allowing the continuous assessment of their physical and emotional state, as implemented through the original SISEI system, which consists of hardware (Tablet, Smart Button for Immediate Interference and Remote Control, Smart Watch, Blood Pressure Monitor, Scale, Oximeter, Activity Tracker) and software (Android App, Web App, Helpdesk App, WebRTC App, Exergame App, Authentication Services, API Services, Admin API Services, Emotion Analysis Services, Video Recording Services, Cron Server, Websocket Server). The surveillance method implemented by the SISEI system incorporates combined monitoring of the patient's vital signs, broad engagement of the patient with the social environment, personalized information on the personal diet, medications and health routines as well as news and articles, assessment of his emotional condition by exploiting innovative computer vision and machine learning technologies, restoration of the physical and mental condition by using serious games like Cognitive - Exergames and increased usability for users with mobility problems. The method includes the following four (4) parts:
i Medical monitoring of the patient, **characterized by** the collection and storage of information concerning the patient's health, as well as the physical and emotional state, in digital format with the aim of creating an electronic personal health file, recording, and storing the bio-signals and the daily of the patient's activity using commercial medical and wearable devices with Bluetooth / BLE connectivity, as well as smart devices with Wi-Fi connectivity, the evaluation of the emotional state through image and video analysis sent to it by user applications to assess the emotional state by reducing it to 6 basic emotions and storing it, the processing, analysis and consideration of all recorded information to identify extreme values and, by extension, dangerous situations and the detection of outliers, based on limits set by the medical staff, and a report edition with the recorded information of the patient and the alarm regarding the detection of extreme values as well as advice and reminders for the received medication.
ii Patient information and entertainment, **characterized by** the sharing of multimedia material with familiar people, automated viewing of domestic and global news, and physical and mental exercise through serious cognitive and exergames.
iii Communication of the patient with the specialized care staff or a familiar person, **characterized by** the use of video calling and other common features of social media, including the exchange of text messages and multimedia materials.
iv Patient emergency response, **characterized by** automated detection of potential dangerous events through automatic evaluation of recorded measurements and notification of medical and/or nursing staff in case of exceeding the physiological limits set by the attending doctor, as well as the easy-to-use "red button" emergency call service, through which the patient can immediately call the nursing staff, who receive the notification through the system on the smart device they use to monitor the patients (tablet / smartphone).

2. The method according to the main claim, by which simplified command is provided for patients with mobility problems or limited experience with PCs, **characterized by** the use of a smart button, which is wireless, capable of communication via Bluetooth / BLE, and incorporates motion detection sensors, as well as the ability to program the press feature, giving the ability of remote control of the terminal with a limited number of movements, through which the basic features of using the tablet can be performed and a quick way to call for help is provided.

3. The method according to the first part of the main claim, by which the collection of information is stored in a database for exploitation, **characterized by** the automated integration of information from heterogeneous devices regarding the supported software, communication protocol and operational specifications.

4. The method according to the first part of the main claim, by which bio signals and daily activity information are collected, using suitable wireless medical and wearable devices, such as a Smart Watch, Sphygmomanometer, Scale, Oximeter, and Activity Tracker, and stored in a database to be exploited.

5. The method according to the first part of the main claim, by which the emotional state of the patient is assessed, **characterized by** the use of machine learning models for the analysis of facial expressions from an image or video collected by the terminal in real time for the extraction of advanced emotion indicators and estimation of the emotional state by reduction to 6 basic emotions: sadness, happiness, fear, anger, surprise and disgust.

6. The method according to the first part of the main claim, with which processing, analysis and consideration of the collected information is performed on a twenty-four-hour basis to identify extreme values and outliers in the derived measurements from the system devices, based on the permissible limits which have been specified in the information system by the medical staff.

7. The method according to the first part of the main claim, with which a report **characterized by** increased visualization capabilities is issued for the patient's bio signals and daily activity.

8. The method according to the first part of the main claim, by which an alarm is issued, **characterized by** an automated call, through the system, to the relevant nursing staff when extreme values are detected.

9. The method according to the first part of the main claim, by which advice and reminders about the received medication are sent at the appropriate time, **characterized by** the personalized recommendations registered by the medical staff, based on the needs and requirements resulting from the analysis of the electronic health record.

10. The method according to the second part of the main claim, by which the remote control of the exergame is performed, characterized using a special camera for locating a part of the body and exploiting its movement for command.

11. The method according to the second part of the main claim, by which performance in serious games, cognitive and exergames, is recorded and stored, based on indicators defined per game with the input of medical personnel.

12. The method according to the third part of the main claim, by which the communication between the patient and the doctor is recorded.

13. The method according to the fourth part of the main claim, by which the potentially dangerous situations are automatically detected by detecting extreme values and the relevant staff is informed or called by the use of the smart button by the patient.
